Europäisches Patentamt

European Patent Office

Office européen des brevets

(11) Veröffentlichungsnummer: **0 463 196 A1**

## EUROPÄISCHE PATENTANMELDUNG

(21) Anmeldenummer: **90111974.3**

(22) Anmeldetag: **23.06.90**

(51) Int. Cl.⁵: **A61B 17/39**

(43) Veröffentlichungstag der Anmeldung:
**02.01.92 Patentblatt 92/01**

(84) Benannte Vertragsstaaten:
**AT DE DK GB**

(71) Anmelder: **Erbe Elektromedizin GmbH.**
**Waldhörnlestrasse 17**
**W-7400 Tübingen(DE)**

(72) Erfinder: **Farin, Günter**
**Kapellenweg 15,**
**W-7400 Tübingen(DE)**

(74) Vertreter: **Endlich, Fritz, Dipl.-Phys.**
**Postfach 1326 Blumenstrasse 8**
**W-8034 Germering(DE)**

(54) **Neutralelektrode für die Hochfrequenzchirurgie.**

(57) Es wird eine Neutralelektrode für die Hochfrequenzchirurgie beschrieben, die eine auf einer Kunststoffplatte angeordnete Metallfolie mit einer Kontaktfläche 1 und eine einstückig damit ausgebildete Kontaktlasche zum Anschluß eines zweipoligen Kabels 3 für einen Überwachungsstromkreis aufweist. Die Kontaktlasche 20 hat zwei derart voneinander getrennte Anschlußstücke 11,12, daß an das eine Anschlußstück 11 nur der erste Anschlußkontakt 6 und an das andere Anschlußstück 12 nur der andere Anschlußkontakt 7 des zweipoligen Kabels 3 anschließbar ist. Die beiden Anschlußstücke können beispielsweise durch einen in die Kontaktfläche 1 hineinragenden mittigen Schlitz 8 in der Metallfolie der Kontaktlasche 20 gebildet sein.

Fig. 4

EP 0 463 196 A1

Die Erfindung betrifft eine Neutralelektrode für die Hochfrequenzchirurgie bestehend aus einer auf einer Kunststoffplatte angeordneten Metallfolie mit einer Kontaktfläche und einer einstückig damit ausgebildeten Kontaktlasche zum Anschluß eines zweipoligen Kabels für einen Überwachungsstromkreis.

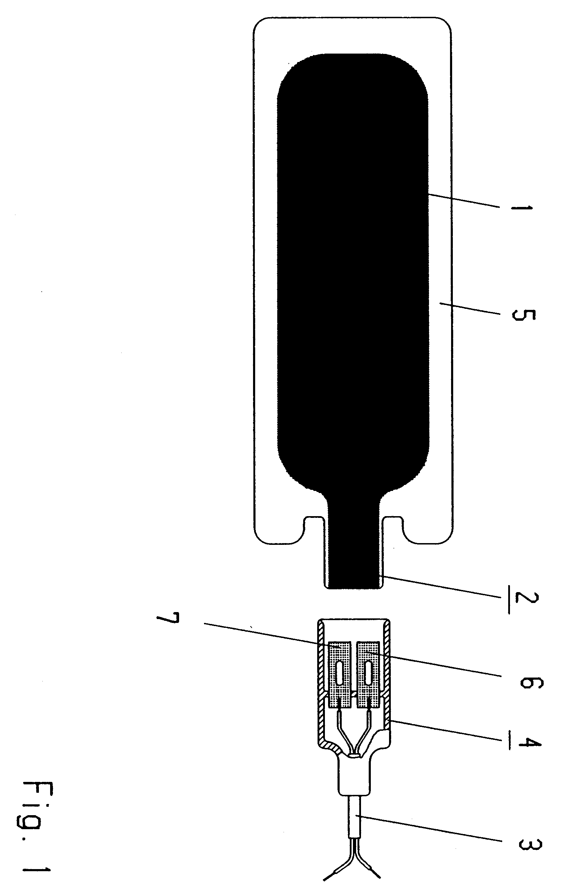

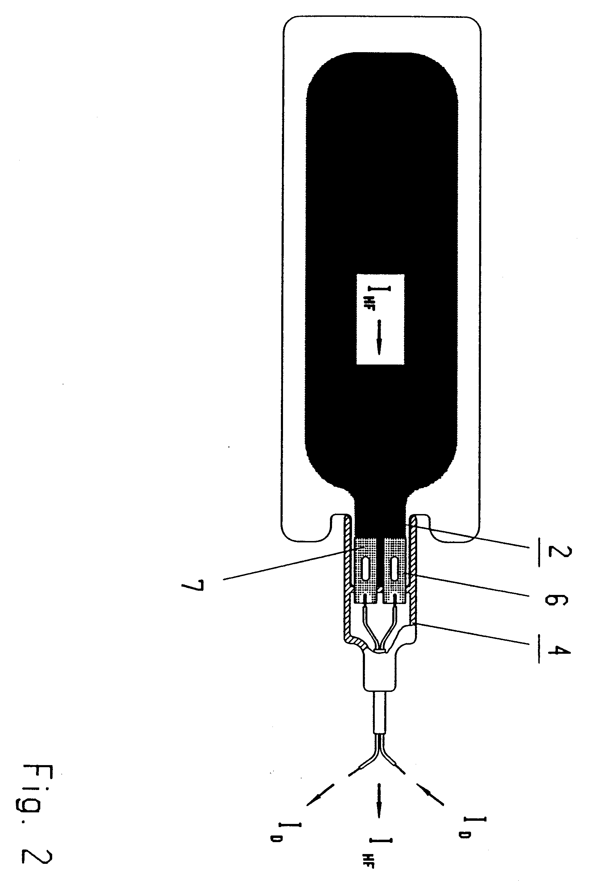

Neutralelektroden werden in der Hochfrequenzchirurgie zum Ableiten des hochfrequenten elektrischen Stroms vom Patienten verwendet. Hierbei muß die Neutralelektrode sowohl mit dem Hochfrequenz-Chirurgiegerät als auch mit dem Patienten elektrisch einwandfrei verbunden sein. Bezüglich der elektrischen Verbindung zwischen Neutralelektrode und Hochfrequenz-Chirurgiegerät schreibt DIN/VDE 0750 Teil 202 für Hochfrequenz-Chirurgiegeräte mit einer Nenn-Ausgangsleistung von mehr als 50 Watt vor, daß diese Geräte mit einem Überwachungsstromkreis ausgerüstet sein müssen, der den Ausgang spannungslos macht und ein akustisches Signal auslöst, wenn eine Unterbrechung der Leitung zur Neutralelektrode oder ihrer Anschlüsse auftritt. Der Überwachungsstrom muß durch einen Teil der Neutralelektrode fließen. Die elektrisch leitfähige Verbindung zwischen Neutralelektrode und deren Anschlußkabel kann auf verschiedene Weisen realisiert werden. Bei Neutralelektroden für einmalige Anwendung wird diese Verbindung mit Rücksicht auf die Herstellkosten bevorzugt durch eine Anschlußlasche realisiert, welche als Verlängerung aus der Kontaktfläche herausgestaltet wird. Eine bekannte Neutralelektrode dieser Art ist schematisch in Fig. 1 dargestellt. Die Kontaktfläche 1 besteht beispielsweise aus einer Metallfolie, die auf einer flexiblen Kunststoffplatte 5 angeordnet ist. Die Kontaktlasche 2 zum Anschluß eines zweipoligen Kabels 3 mittels einer zweipoligen Kontaktklammer 4 wird beispielsweise durch Verlängern der Metallfolie und der Kunststoffplatte 5 aus der Kontaktfläche 1 herausgeführt. Wird die Kontaktklammer 4, wie in Fig. 2 dargestellt, auf die Kontaktlasche 2 gesteckt, so überbrückt die Metallfolie im Bereich der Kontaktlasche 2 die beiden Kontakte 6,7 und der Überwachungsstrom $I_D$ fließt durch die Kontaktlasche 2.

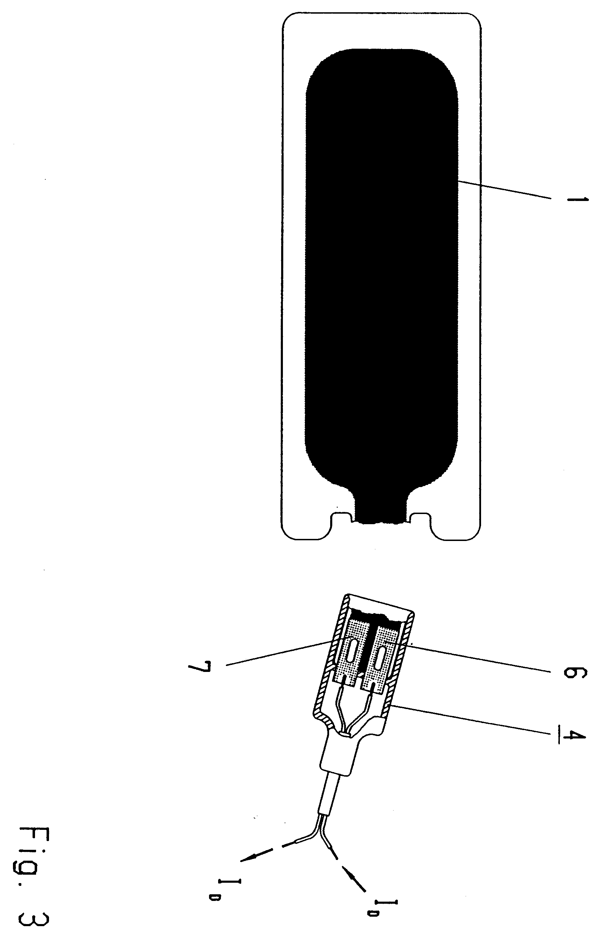

Da die Metallfolie mit Rücksicht auf die Kosten und die Flexibilität der Neutralelektrode einerseits möglichst dünn sein sollte, besteht andererseits das Problem, daß sie bei mechanischer Belastung, beispielsweise bei einem Zug an der Kontaktklammer 4, wie in Fig. 3 schematisch dargestellt, im Bereich zwischen den Kontakten 6,7 und der Kontaktfläche 1 auseinanderreißt, so daß der hochfrequente Strom $I_{HF}$ nicht mehr von der Kontaktfläche 1 zum Hochfrequenz-Chirurgiegerät fließen kann. Da der Überwachungsstrom $I_D$ durch die abgerissene Metallfolie innerhalb der Kontaktklammer weiter fließen kann, wird ein derartiger Fehler durch den Überwachungsstromkreis nicht erkannt.

Es sind Verbrennungen an Patienten bekannt geworden, welche dadurch verursacht waren, daß die elektrische Verbindung zwischen der Kontaktlasche und der Anschlußlasche von Neutralelektroden unterbrochen war und der hochfrequente elektrische Strom entweder durch den kleinen Kontakt zwischen abgerissener Lasche und Patient oder durch andere elektrisch leitfähige Teile geflossen ist, mit denen der Patient Kontakt hatte.

Es ist deshalb Aufgabe der Erfindung, eine Neutralelektrode für die Hochfrequenzchirurgie derart zu verbessern, daß Schwierigkeiten der genannten Art bei einer für eine Massenproduktion geeigneten Neutralelektrode für eine einmalige Anwendung möglichst weitgehend vermieden werden können.

Diese Aufgabe wird erfindungsgemäß durch den Gegenstand des Patentanspruchs 1 gelöst. Zweckmäßige Ausgestaltungen der Erfindung sind Gegenstand der Unteransprüche.

Ein besonderer Vorteil der Erfindung ist darin zu sehen, daß ein Abriß der Metallfolie im Bereich der Kontaktlasche, der zu Verbrennungen am Patienten führen könnte, durch den Überwachungsstromkreis sofort erfaßt wird, so daß beispielsweise eine Warnanzeige oder eine automatische Abschaltung des Hochfrequenz-Chirurgiegeräts verursacht werden kann.

Anhand der Zeichnung soll die Erfindung beispielsweise näher erläutert werden. Es zeigen:

Fig. 1 bis 3
eine schematische Darstellung einer bekannten einflächigen Neutralelektrode mit einer Kontaktlasche, mit der eine zweipolige Kontaktklammer in Verbindung gebracht werden kann,

Fig. 4
eine Darstellung einer einflächigen Neutralelektrode gemäß der Erfindung,

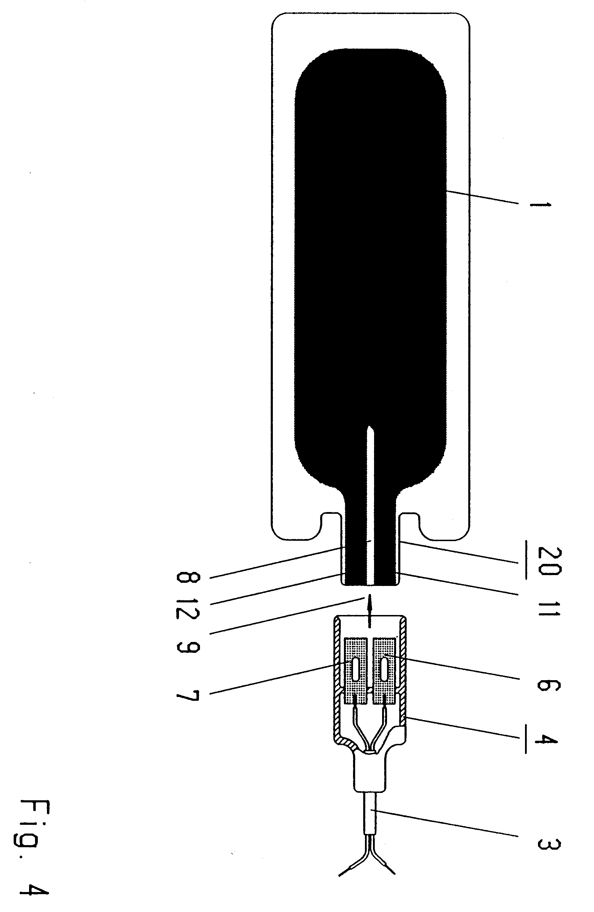

Fig. 5
die Neutralelektrode in Fig. 4, wenn die Kontaktlasche von der Kontaktfläche abgerissen wird; und

Fig. 6 bzw.Fig. 7
zwei weitere Ausführungsbeispiele einer Neutralelektrode gemäß der Erfindung.

Fig. 1 zeigt in schematischer Darstellung eine bekannte einflächige Neutralelektrode mit einer einpoligen Kontaktlasche. Die Kontaktfläche 1 besteht beispielsweise aus einer Metallfolie, die auf einer flexiblen Kunststoffplatte 5 angeordnet ist. Die Kontaktlasche 2 zum Anschluß einer zweipoligen Kabels 3 mittels einer zweipoligen Kontaktklammer 4 wird beispielsweise durch Verlängern der Metallfolie und der Kunststoffplatte aus der Kontaktfläche 1 herausgeführt. Wird die zweipolige Kontaktklammer 4, wie in Fig. 2 dargestellt, auf die einpolige Kontaktlasche 2 gesteckt, so überbrückt die Metallfolie der Kontaktlasche 2 die beiden Kontakte 6 und 7 und der Überwachungsstrom $I_D$ fließt durch die Kontaktlasche 2. Reißt die Metallfolie, wie in Fig. 3 schematisch dargestellt, beispielsweise infolge einer mechanischen Belastung im Bereich zwischen den Kontakten 6,7 der Kontaktklammer 4 und der Kontaktfläche 1, so daß die elektrische Verbindung zwischen der Kontaktklammer 4 und der Kontaktfläche 1 unterbrochen ist, so kann der hochfrequente Strom $I_{HF}$ nicht zum Hochfrequenz-Chirurgiegerät fließen. Da der Überwachungsstrom $I_D$ trotz einer derartigen Unterbrechung weiter durch den abgerissenen Teil der Metallfolie zwischen den Kontakten 6,7 der Kontaktklammer fließt, kann der Überwachungsstromkreis derartige Fehler nicht erfassen.

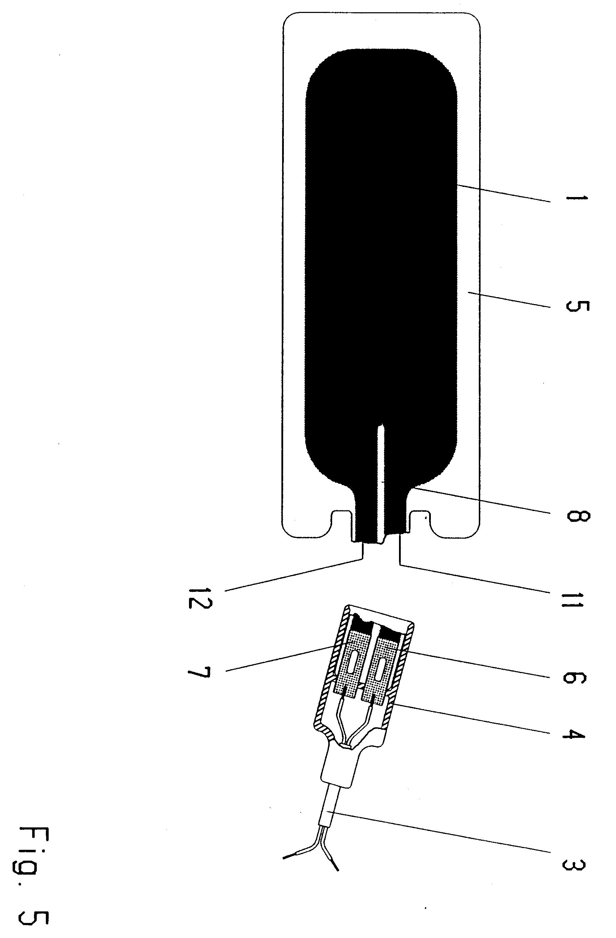

Fig. 4 zeigt in schematischer Darstellung ein Ausführungsbeispiel einer erfindungsgemäßen einflächigen Neutralelektrode, bei welcher die Kontaktlasche 2 in Fig. 1 - 3 als zweipolige Kontaktlasche 20 ausgeführt ist. Die Zweipoligkeit kann in einfacher Weise durch einen elektrisch isolierenden Schlitz 8 realisiert werden, der bei Verwendung einer Kontaktklammer in Steckrichtung 9 der Kontaktklammer 4 beispielsweise mittig durch die Metallfolie der Kontaktlasche 20 und mehr oder weniger weit in die Kontaktfläche 1 hineingeführt ist. Eine derartige Kontaktlasche 20 weist deshalb zwei derart voneinander getrennte Anschlußstücke 11,12 auf, daß an das eine Anschlußstück 11 nur der eine Anschlußkontakt 6 und an das andere Anschlußstück 12 nur der andere Anschlußkontakt 7 des zweipoligen Kabels 3 anschließbar ist. Die Anschlußkontakte 6,7 die entsprechend dem dargestellten bevorzugten Ausführungsbeispiel an einer Kontaktklammer vorgesehen sind, können auch unmittelbar an einer zweiadrigen Anschlußleitung vorgesehen sein, die ebenfalls nur für eine einmalige Anwendung vorgesehen ist. Reißt die Metallfolie bei einer derartig gestalteten Neutralelektrode, wie in Fig. 5 schematisch dargestellt, im Bereich zwischen den Anschlußkontakten 6,7 der Kontaktklammer 4 und der Kontaktfläche 1, so kann der Überwachungsstrom $I_D$ nicht mehr fließen.

Für die Produktion einer derartigen Neutralelektrode kann es zweckmäßig sein, den Schlitz 8 z.B. in einem einzigen Arbeitsgang sowohl in die Metallfolie als auch in die Kunststoffplatte 5 zu stanzen.

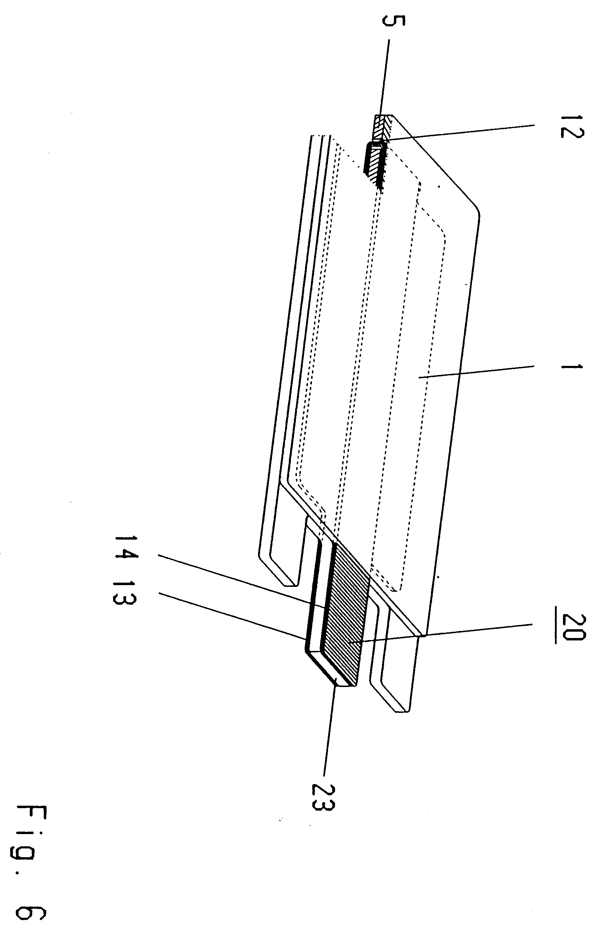

Die Zweipoligkeit der Kontaktlasche 20 der einflächigen Neutralelektrode kann auch auf andere Weise realisiert werden. Wie in Fig. 6 dargestellt, kann ein Metallfolien-Streifen 13 als Verlängerung der Kontaktfläche 1 auf der Kontaktseite der Neutralelektrode und ein zweiter Metallfolien-Streifen 14, der auf der Rückseite der Kunststoffplatte 5 angeordnet ist und der im Bereich der Kontaktfläche 1 durch einen Schlitz 12 durch die Kunststoffplatte 5 hindurch mit der Metallfolie der Kontaktfläche 1 elektrisch leitfähig kontaktiert ist, realisiert werden. Die Isolation 23 zwischen den Metallfolien-Streifen 13,14 kann aus der Kunststoffplatte 5 herausgeformt werden. Hierbei wird die Kontaktklammer 4, welche in Fig. 6 nicht dargestellt ist, bezüglich der Anordnung der Anschlußkontakte 6,7 derart gestaltet, daß die beiden Anschlußkontakte nicht nebeneinander, wie in Fig. 1 dargestellt, sondern auf gegenüberliegenden Seiten der Kontaktklammer angeordnet sind.

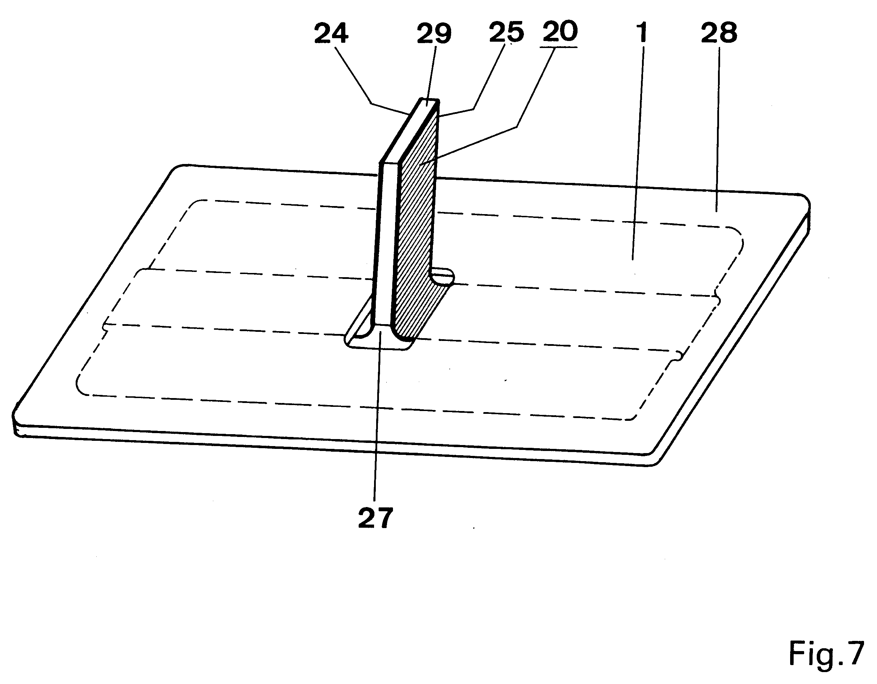

Fig. 7 zeigt ein weiteres Ausführungsbeispiel, bei dem zwei elektrisch durch eine Isolationsschicht 29 voneinander isolierte Metallfolien-Streifen 24,25 vorgesehen sind, die mit der Kontaktfläche 1 elektrisch leitfähig verbunden sind. Durch eine schlitzförmige Öffnung 27 in der Kunststoffplatte 28, die beispielsweise mittig auf der Rückseite der Neutralelektrode vorgesehen ist, sind die Metallfolienstreifen 24,25 nach außen durchgeführt, um die Kontaktlasche 20 zu bilden. Die Metallfolienstreifen 24,25 können beispielsweise auf der Rückseite der Kontaktfläche 1 der Neutralelektrode durch Löten oder Schweißen angebracht sein, oder als Verlängerung aus dieser Kontaktfläche herausgeformt und durch die schlitzförmige Öffnung 27 hindurchgeführt sein. Bei diesem Ausführungsbeispiel ergibt sich der Vorteil, daß ein Zug an der Kontaktlasche 20 nicht so leicht zum Abschälen der Neutralelektrode von der Haut des Patienten führen kann, wie es bei den in den Fig. 4 bis 6 dargestellten Ausführungsbeispielen und auch bei den in den Fig. 1 bis 3 dargestellten bekannten Elektroden der Fall ist, bei denen die Kontaktlasche an einem Endteil der Kontaktfläche 1 angeordnet ist.

**Patentansprüche**

1. Neutralelektrode für die Hochfrequenzchirurgie bestehend aus einer auf einer Kunststoffplatte (5) angeordneten Metallfolie mit einer Kontaktfläche (1) und einer einstückig damit ausgebildeten Kontaktlasche zum Anschluß eines zweipoligen Kabels (3) für einen Überwachungsstromkreis, **dadurch gekennzeichnet,** daß die Kontaktlasche (20) zwei derart voneinander getrennte Anschlußstücke (11,12; 13,14;24,25) aufweist, daß an das eine Anschlußstück (11) nur der eine Anschlußkontakt (6) und an das andere Anschlußstück (12) nur der andere Anschlußkontakt (7) des zweipoligen Kabels (3) anschließbar ist.

2. Neutralelektrode nach Anspruch 1, **dadurch gekennzeichnet,** daß die beiden Anschlußkontakte (6,7) nebeneinander an einer Kontaktklammer (4) vorgesehen sind.

3. Neutralelektrode nach Anspruch 1 oder 2, **dadurch gekennzeichnet,** daß die beiden Anschlußstücke (11,12) durch einen in die Kontaktfläche (1) hineinreichenden mittigen Schlitz (8) in der Metallfolie der Kontaktlasche (20) gebildet sind.

4. Neutralelektrode nach Anspruch 1 oder 3, **dadurch gekennzeichnet,** daß die beiden Anschlußstücke (11,12) durch einen gemeinsamen Schlitz (8) in der Metallfolie und in der Kunststoffplatte (5) gebildet sind.

5. Neutralelektrode nach Anspruch 1, **dadurch gekennzeichnet,** daß die beiden Anschlußendstücke durch zwei übereinander angeordnete, durch eine Isolationsschicht (23) voneinander isolierte Metallfolienstreifen (13,14) gebildet sind, die mit zwei gegenüberliegenden Endteilen der Kontaktfläche elektrisch leitend verbunden sind, und daß die dadurch gebildete Kontaktlasche (20) von dem einen Endteil der Kontaktfläche vorragt.

6. Neutralelektrode nach Anspruch 1, **dadurch gekennzeichnet,** daß die beiden Anschlußendstücke durch zwei übereinander angeordnete, durch eine Isolationsschicht (29) voneinander isolierte Metallfolienstreifen (24,25) gebildet sind, die mit zwei gegenüberliegenden Endteilen der Kontaktfläche elektrisch leitend verbunden sind, und daß die beiden durch eine Isolationsschicht (29) voneinander isolierten Metallfolienstreifen (24,25) durch eine zentrale Öffnung (27) in der Kunststoffplatte vorragen, so daß die Kontaktlasche (20) von einer zentralen Stelle auf der der Kontaktfläche (1) gegenüberliegenden Seite vorragt.

7. Neutralelektrode nach Anspruch 5 oder 6, **dadurch gekennzeichnet,** daß die beiden Anschlußkontakte (6,7) auf gegenüberliegenden Seiten an einer Kontaktklammer vorgesehen sind.

Fig. 1

Fig. 2

$I_{HF}$

7

2

6

4

$I_D$

$I_{HF}$

$I_D$

Fig. 3

EP 0 463 196 A1

Fig. 4

Fig. 5

Fig. 6

Fig.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | GB-A-1 601 588  (DRG LTD.)<br>* Ansprüche 1,8; Seite 2, Zeilen 52-68; Figur 2 *<br>– – – | 1 | A 61 B 17/39 |
| Y | DE-A-3 364 67  (R. HEILBRUN)<br>* Ansprüche 1,2; Figuren 1,2 *<br>– – – | 1 | |
| A | US-A-4 722 761  (J.V. CARTMELL et al.)<br>* Anspruch 1; Spalte 3, Zeilen 31-39; Figur 1 *<br>– – – | 1-5 | |
| A | DE-A-3 544 483  (P. FEUCHT)<br>* Ansprüche 1,2; Zusammenfassung *<br>– – – | 1-4 | |
| A | US-A-4 237 886  (I. SAKURADA et al.)<br>* Ansprüche 11-15; Figur 2 *<br>– – – – – | 6 | |

| | RECHERCHIERTE SACHGEBIETE (Int. Cl.5) |
|---|---|
| | A 61 B |

Der vorliegende Recherchenbericht wurde für alle Patentansprüche erstellt

| Recherchenort | Abschlußdatum der Recherche | Prüfer |
|---|---|---|
| Berlin | 04 Dezember 90 | PAPA E.R. |